# EUROPÄISCHE PATENTSCHRIFT

(11) **EP 3 723 670 B1**
(45) Veröffentlichungstag und Bekanntmachung des Hinweises auf die Patenterteilung: **16.06.2021**
(21) Anmeldenummer: 18826236.4
(22) Anmeldetag: 13.12.2018
(51) Int. Cl.: A61F 2/78

(54) **PROTHESENSCHAFTSYSTEM**
PROSTHESIS SYSTEM
SYSTÈME DE PROTHÈSE

(30) Priorität: 14.12.2017 DE 102017129930
(43) Veröffentlichungstag der Anmeldung: 21.10.2020
(73) Patentinhaber: Ottobock SE & Co. KGaA, 37115 Duderstadt (DE)
(72) Erfinder: VOLKMAR, Jens, 37115 Duderstadt (DE); GOTTLIEB, Harald, 37345 Sonnenstein (DE); LEINIGER, Andreas, 37327 Leinefeld (DE); BULS, Sebastian, 37115 Duderstadt (DE)
(74) Vertreter: Gramm, Lins & Partner Patent- und Rechtsanwälte PartGmbB
(86) Internationale Anmeldenummer: PCT/EP2018/084726
(87) Internationale Veröffentlichungsnummer: WO 2019/115681

(56) Entgegenhaltungen:
- WO-A1-2017/189398
- DE-A1-102006 046 928
- DE-A1-102011 117 801
- US-A1- 2017 056 212

## Beschreibung

Die Erfindung betrifft ein Prothesenschaftsystem mit einem starren Außenschaft, der eine proximale Außenschaftöffnung zur Aufnahme eines Extremitätenstumpfes eines Trägers des Prothesenschaftsystemes aufweist, und einem flexiblen Innenschaft, der mit dem Außenschaft verbunden ist und eine proximale Innenschaftöffnung zur Aufnahme des Extremitätenstumpfes aufweist.

Ein derartiges Prothesenschaftsystem ist beispielsweise aus der DE 10 2011 117 801 A1 bekannt. Prothesenschaftsysteme dieser Art werden häufig als Beinprothesen ausgestaltet, beispielsweise für Patienten mit transtibialer oder transfemoraler Amputation. Damit das Prothesenschaftsystem fest und sicher an den Extremitätenstumpf seines Trägers angelegt werden kann, wird zwischen dem Innenschaft und dem Extremitätenstumpf üblicherweise ein Unterdruck erzeugt. Der Vorteil derartiger Prothesenschaftsysteme gegenüber unflexiblen Prothesenschäften ist, dass Schwankungen des Volumens des Extremitätenstumpfes aufgefangen werden können, weil eine Anpassung von dem flexiblen Innenschaft an den Extremitätenstumpf erfolgt. Aufgrund des flexiblen Innenschaftes ergeben sich jedoch andere Herausforderungen für das Anlegen des Prothesenschaftsystemes an den Extremitätenstumpf. Es ist wünschenswert, einen festen, sicheren und komfortablen Halt mit einfachen konstruktiven Mitteln zu erreichen.

Aus der US 2017/0056212 A1 ist ein System aus Prothesenschaft und Prothesenliner bekannt, wobei der Prothesenliner auf seiner dem Prothesenträger zugewandten Oberfläche proximal einen Bereich mit geringer oder keiner Texturierung aufweist. Hierdurch soll eine gute Abdichtung zwischen Prothesenliner und der Haut des Prothesenträgers in diesem Bereich erzielt werden.

Der Erfindung liegt daher die Aufgabe zugrunde, ein Prothesenschaftsystem bereitzustellen, bei dem ein sicherer Halt des Extremitätenstumpfes eines Patienten in dem Innenschaft des Prothesenschaftsystemes mit einfachen konstruktiven Mitteln und gleichzeitig bei einem hohen Tragekomfort bereitgestellt wird.

Die Erfindung löst die gestellte Aufgabe durch ein Prothesenschaftsystem mit den Merkmalen des Hauptanspruches. Vorteilhafte Ausgestaltungen und Weiterbildungen der Erfindung sind in den Unteransprüchen, der Beschreibung und den Figuren offenbart.

Das Prothesenschaftsystem mit einem starren Außenschaft, der eine proximale Außenschaftöffnung zur Aufnahme eines Extremitätenstumpfes eines Trägers des Prothesenschaftsystemes aufweist, und einem flexiblen Innenschaft, der mit dem Außenschaft verbunden ist und eine proximale Innenschaftöffnung zur Aufnahme des Extremitätenstumpfes aufweist, sieht vor, dass zumindest eine Dichtlippe an einer dem Extremitätenstumpf zugewandten Innenseite des Innenschaftes angeordnet ist.

Durch die Dichtlippe wird ein Raum zwischen dem Innenschaft und dem Extremitätenstumpf abgedichtet. Wenn ein Unterdruck zwischen dem Extremitätenstumpf und dem Innenschaft angelegt wird, dient die Dichtlippe dem Abdichten des Innenraumes zwischen Innenschaft und Extremitätenstumpf gegenüber der Umgebung. Die Dichtlippe wird dabei in Richtung des Unterdruckes gezogen und legt sich an dem Extremitätenstumpf an. Bislang war es üblich, eine gelenkübergreifende Abdichtung, beispielsweise eine Kniekappe, über das Prothesenschaftsystem und den Extremitätenstumpf zu stülpen, um den Innenschaft zusammen mit dem Au-ßenschaft gegenüber der Umgebung abzudichten und einen besseren Halt des Innenschaftes an dem Extremitätenstumpf mittels Unterdruckes zu bewerkstelligen. Mit dem erfindungsgemäßen Prothesenschaftsystem ist eine sichere Festlegung des Prothesenschaftsystems an dem Patienten weiterhin möglich, eine den Au-ßenschaft umgreifende Manschette jedoch nicht mehr notwendig, weil eine hinreichende Unterdruckfixierung bereits mit der Dichtlippe bereitgestellt werden kann. Das erfindungsgemäße Prothesenschaftsystem kann daher besonders einfach und schnell angelegt werden. Es kann mit und ohne Prothesenliner getragen werden. Mit dem erfindungsgemäßen Prothesenschaftsystem können sowohl ovale als auch unregelmäßige Schaftquerschnitte abgedichtet werden. Es eignet sich in gleichem Maße für transkarpale und transradiale Stümpfe.

Der starre Außenschaft ist vorzugsweise zu einem überwiegenden oder vollständigen Teil aus einem starren Material, beispielsweise einem faserverstärkten Kunststoff, insbesondere einem kohlefaser- oder glasfaserverstärkten Kunststoff, ausgebildet. Diese Materialien sind leicht zu bearbeiten, sind stabil und weisen dennoch ein sehr geringes Eigengewicht auf. Auf diese Weise ist ein besonders leichter Prothesenschaft herstellbar, der über gute Stabilitätseigenschaften verfügt. Insbesondere durch die hohe Eigenstabilität des Außenschaftes wird dem Träger des erfindungsgemäßen Prothesenschaftsystemes ein Gefühl hoher Sicherheit vermittelt. Durch die hohe Stabilität und Festigkeit des Außenschaftes ist zudem eine sichere und stabile Anordnung distaler Protheseneinrichtungen, wie beispielsweise eines Prothesenfußes oder eines Prothesenkniegelenks mit Unterschenkelrohr und Prothesenfuß, möglich.

Der flexible Innenschaft ist vorzugsweise überwiegend oder vollständig aus einem flexiblen Material hergestellt. Bevorzugt weist das flexible Material gute Polsterungseigenschaften und elastische Eigenschaften auf. Als flexibles Material für den Innenschaft wird vorzugsweise ein Silikon, beispielsweise ein HTV-Silikon, verwendet, welches eine hohe Atmungsaktivität aufweist und keine Funktionseinschränkung der Muskulatur des Extremitätenstumpfes verursacht. Die Elastizität des Silikons kann nach Bedarf durch die Einarbeitung eines Gewebes eingeschränkt werden. Zusätzlich oder alternativ kann der flexible Innenschaft eine Polsterung auf seiner Innenseite aufweisen. Die Polsterung kann im Wesentlichen auf der gesamten Fläche oder einer Teilfläche der Innenseite des Innenschaftes, gegebenenfalls mit Ausnahme von Bereichen um die Dichtlippe herum, angeordnet sein. Alternativ oder zusätzlich kann die Polsterung auf bestimmten Zonen der Innenseite des Innenschaftes angeordnet sein. Beispielsweise können nur Zonen oder zusätzlich solche Zonen, in denen ein besonders hoher Druck bei der Anlage des Innenschaftes auf den Extremitätenstumpf wirkt, gepolstert werden.

Die Verbindung des Innenschaftes mit dem Außenschaft ist vorzugsweise eine überwiegend durch Formschluss und/oder Kraftschluss hergestellte, bevorzugt lösbare, Verbindung, beispielsweise eine Schraubverbindung oder ein Verbindung mittels Klettverschluss oder Verhakungselementen. Zusätzlich oder alternativ ist auch eine stoffschlüssige Verbindung des Innenschaftes mit dem Außenschaft möglich.

Erfindungsgemäß ist zumindest eine Dichtlippe an einer dem Extremitätenstumpf zugewandten Innenseite des Innenschaftes angeordnet. Es können jedoch auch zwei, drei, vier und mehr Dichtlippen an der Innenseite des Innenschaftes angeordnet sein. Mehrere Dichtlippen können unmittelbar nebeneinander und/oder in Richtung einer proximal-distalen Erstreckung des Innenschaftes und/oder entlang eines Innenumfanges des Innenschaftes voneinander beabstandet angeordnet sein.

In einer Weiterbildung der Erfindung ist die Dichtlippe als eine umlaufende Dichtlippe ausgebildet, die entlang eines gesamten Innenumfanges der Innenseite des Innenschaftes angeordnet ist. Dies hat den Vorteil, dass eine vollständige Abdichtung mit nur einer Dichtlippe entlang des gesamten Innenumfanges des Innenschaftes und somit des gesamten Umfanges des Extremitätenstumpfes erfolgen kann. Es können auch mehrere umlaufende Dichtlippen entlang der proximal-distalen Erstreckung des Innenschaftes zueinander beabstandet angeordnet sein. Dies kann zusätzlichen Halt bewirken, beispielsweise wenn der Extremitätenstumpf in Richtung seiner proximal-distalen Erstreckung relativ ungleichförmig ist. Wenn beispielsweise der Extremitätenstumpf entlang seines Umfanges nicht vollständig von einer ersten umlaufenden Dichtlippe umschlossen wird, so dass der notwendige Unterdruck zum sicheren Halten des Innenschaftes nicht hergestellt werden kann, kann der Unterdruck für einen sicheren Halt noch immer durch eine zweite oder dritte oder weitere umlaufende Dichtlippen erzeugt werden, die distal oder proximal von der ersten umlaufenden Dichtlippe angeordnet sein können, je nachdem in welche Richtung oder Richtungen ein Unterdruck wirkt. Zusätzlich ist es möglich, eine oder mehrere Unterbrechungen in Form von Schlitzen in oder Materialausnehmungen der Dichtlippe in dem Verlauf der umlaufenden Dichtlippe vorzusehen, um eine Entlastung der auf den Extremitätenstumpf wirkenden Kräfte durch eine andere Druckverteilung zu bewirken.

Gemäß einer Weiterbildung der Erfindung ist in dem Außenschaft zumindest ein Fenster, insbesondere zur Entlastung eines auf den Extremitätenstumpf ausgeübten Druckes, ausgebildet. Ein solches Fenster reduziert das Gewicht des Außenschaftes und gibt dem Träger des Prothesenschaftsystemes die Möglichkeit, in diesem Bereich Einflüsse der Außenwelt wahrzunehmen. Insbesondere ist zumindest ein Fenster ausgebildet, bevorzugt sind jedoch zwei, drei, vier oder mehr Fenster in dem Außenschaft ausgebildet. Die Fenster sind vorteilhafterweise an die Anatomie der zu versorgenden Extremität bzw. des Extremitätenstumpfes, wie z.B. an Knochen oder Muskulatur, angepasst. Das zumindest eine Fenster ist bevorzugt mindestens teilweise, insbesondere vollständig, durch den flexiblen Innenschaft ausgefüllt. Vorzugsweise ist der flexible Innenschaft hierfür bis auf seine proximale Innenschaftöffnung und mögliche Ausnehmungen, beispielsweise für die Verbindung mit dem Außenschaft oder eine Aufnahme eines Ausstoßventiles oder eines Sauganschlusses, vollständig geschlossen ausgebildet. Insbesondere ist es bevorzugt, wenn der Innenschaft und der Außenschaft in einem distalen Bereich durch jeweils eine Kappe geschlossen sind.

In einer Weiterbildung der Erfindung ist der Außenschaft als Unterschenkelschaft ausgebildet, wobei ein Fenster in einem frontalen Schienbeinbereich und/oder einem distalen Wadenbeinbereich und/oder im Bereich eines Wadenbeinköpfchens angeordnet sein kann. Bei einer Ausgestaltung des Außenschaftes als Unterschenkelschaft kann das Problem auftreten, dass knöcherne Strukturen gegen das Prothesenschaftsystem drücken. Auch wenn der Innenschaft aus einem vergleichsweise weichen, flexiblen und bevorzugt elastischen Material ausgebildet ist, ergeben sich durch den rigiden Außenschaft Anlagestellen, die im Bereich der knöchernen Strukturen zu Schmerzen führen können. Um eine Druckentlastung in diesen Bereichen zu ermöglichen, ist vorgesehen, dass in den Bereichen mit exponierten knöchernen Strukturen ein Fenster vorgesehen ist. So kann beispielsweise in einem frontalen Schienbeinbereich, einem distalen Wadenbeinbereich und/oder in dem Bereich des Wadenbeinköpfchens ein Fenster angeordnet sein, um dort lokal eine Druckentlastung zu ermöglichen. Ebenfalls kann in beiden Bereichen ein entsprechendes Fenster ausgebildet sein. Das Prothesenschaftsystem kann auch zur Aufnahme eines Oberschenkelstumpfes oder eins Stumpfes der oberen Extremität ausgebildet sein. Bei einem Oberschenkelschaft dient das Fenster oder dienen die Fenster als Ausweichmöglichkeit für Volumenschwankungen, knöcherne Strukturen sind an dem Umfang des Außenschaftes nicht zu erwarten. Bei einem Prothesenschaftsystem für die obere Extremität können Fenster in dem Bereich prominenter knöcherner Strukturen angeordnet sein, beispielsweise im Ellenbogenbereich.

Ergänzend kann in einem Wadenbereich des Außenschaftes ein Fenster angeordnet sein, um eine Druckentlastung auch in diesem Bereich zu ermöglichen.

In einer Weiterbildung der Erfindung ist es vorgesehen, dass in zumindest einem der Fenster ein verstellbarer Einsatz angeordnet ist, der an dem Außenschaft befestigt ist. Ein solcher Einsatz kann die Kontur des Außenschaftes aufnehmen und im Wesentlichen der Kontur des Fensters folgen. Auf der Innenseite des Einsatzes, also auf derjenigen Seite, die dem Innenschaft zugewandt ist, kann ein Polster angeordnet sein, so dass bei einer Anordnung des Einsatzes in dem Fenster und einer entsprechenden Befestigung, beispielsweise über einen Gurt, ein Kabelsystem, über Schnallen oder ähnliches, der Einsatz im Wesentlichen die Außenkontur des Außenschaftes ergänzt und darüber hinaus Druck auf den Innenschaft ausübt. Ein Polster ist insbesondere bei Einsetzen Einsätzen von Fenstern in denjenigen Bereichen vorgesehen, die keine knöchernen Strukturen aufweisen, beispielsweise bei einem Oberschenkelschaft an einer nahezu beliebigen Stelle, da das Weichteilgewebe den Oberschenkelknochen nahezu vollständig umgibt. Bei einem Unterschenkelschaft wäre eine Ausstattung mit einem Polster an dem Einsatz im Bereich der Wadenmuskulatur sinnvoll, um Volumenschwankungen ausgleichen zu können und beispielsweise im Verlauf eines Tages bei einem Anschwellen des Stumpfes eine Druckentlastung in dem Weichteilbereich zu ermöglichen oder bei einem Abschwellen des Stumpfes durch Nachstellen und Anziehen einer Spanneinrichtung einen erhöhten Druck auf den Innenschaft und damit auf das Weichteilgewebe ausüben zu können. Dadurch kann ein erhöhtes Sicherheitsempfinden für den Patienten bereitgestellt werden.

Nach einer Weiterbildung der Erfindung ist die Dichtlippe zumindest teilweise in einem Bereich des Innenschaftes angeordnet, der innerhalb des Fensters liegt, also in dem der Innenschaft nicht von dem Außenschaftmaterial abgedeckt ist. Weil die Dichtlippe in dem Bereich eines Fensters eine hohe Flexibilität aufweist, kann sich die Dichtlippe einfacher und komfortabler an den Extremitätenstumpf anlegen, ohne dass ein hoher Druck auf den Stumpf ausgeübt wird. Um eine erhöhte Dichtwirkung zu erzielen, ist die Dichtlippe in denjenigen Bereichen des Innenschaftes angeordnet, die von Außenschaftmaterial überdeckt sind, so dass der Innenschaft mit der Dichtlippe nicht nach außen ausweichen kann

In einer Weiterbildung der Erfindung ist die Dichtlippe näher an der proximalen Innenschaftöffnung als einem distalen Ende des Innenschaftes angeordnet. Eine Ausführungsform der Erfindung sieht vor, dass die Dichtlippe in einem proximalen Abschnitt des Innenschaftes an einer Position angeordnet ist, die gemessen ab der proximalen Innenschaftöffnung in Richtung eines distalen Endes des Innenschaftes ein Drittel oder ein Viertel der Länge des Innenschaftes von der proximalen Innenschaft-öffnung zu dem distalen Ende des Innenschaftes beträgt. Dies hat den Vorteil, dass eine große Anlage- und somit Haftfläche des Extremitätenstumpfes an den Innenschaft zur Verfügung steht. Dadurch kann ein besonders sicherer Halt erzielt werden.

In einer Weiterbildung der Erfindung ist die Dichtlippe in einer Ebene angeordnet und hat somit einen geraden Verlauf, der zu einer gewählten Bezugsrichtung, zum Beispiel der Gravitationsrichtung in der Ausgangsstellung des Prothesenschaftes auch geneigt sein kann, also nicht orthogonal zu der Gravitationsrichtung ausgerichtet ist. Diese Ebene kann beispielsweise quer, insbesondere orthogonal, zu einer proximal-distalen Erstreckung des Innenschaftes verlaufen. Der Extremitätenstumpf wird hierdurch an seinem Umfang entlang einer Ebene abgedichtet.

In einer Weiterbildung der Erfindung ist die Dichtlippe zumindest teilweise parallel zu einer Kontur eines die Innenschaftöffnung umgebenden Innenschaftrandes oder einer Kontur eines die Außenschaftöffnung umgebenden Außenschaftrandes angeordnet. Dies ist besonders bevorzugt für eine in Form einer Raumkurve ausgebildete Kontur des Innenschaftrandes oder Außenschaftrandes. Eine beispielhafte Raumkurve kann im ventralen und dorsalen Bereich je einen Tiefpunkt und im lateralen und medialen Bereich je einen Hochpunkt aufweisen. Durch eine derartige Kontur werden im proximalen Bereich des Prothesenschaftsystemes eine Entlastung im ventralen und dorsalen Bereich und eine Verstärkung im medialen und lateralen Bereich erzielt. Durch die Ausbildung der Dichtlippe parallel zu dieser Kontur wird der Abstand von der Dichtlippe zu der Kontur in vorteilhafter Weise konstant gehalten. Eine derartige Dichtlippe kann außerdem eine Ungleichmäßigkeit des Stumpfvolumens besser ausgleichen und das Tragegefühl verbessern.

In einer Weiterbildung der Erfindung ist die Dichtlippe stoffschlüssig mit dem Innenschaft verbunden oder daran angeformt und/oder die Dichtlippe und der Innenschaft bestehen aus demselben Material. Dies ermöglicht eine leichte Fertigung und hohe Stabilität. Alternativ ist die Dichtlippe formschlüssig und insbesondere auswechselbar an dem Innenschaft befestigt, insbesondere in einer umlaufenden Nut eingelegt.

Vorteilhafterweise unterschieden sich die Materialstärken der Dichtlippe und des Innenschaftes. Eine Materialstärke der Dichtlippe beträgt bevorzugt 5% bis 40%, insbesondere bevorzugt 10% bis 30%, der Materialstärke des Innenschaftes. Dadurch wird eine höhere Flexibilität der Dichtlippe gegenüber dem Innenschaft bereitgestellt, damit diese sich gut an den Extremitätenstumpf anlegen kann.

In einer Weiterbildung der Erfindung weist das Prothesenschaftsystem ein Ausstoßventil und/oder einen Sauganschluss zur Regulierung eines Unterdruckes zwischen dem Extremitätenstumpf und dem Innenschaft auf. Hierfür sind vorzugsweise Ausnehmungen in dem Innenschaft und Außenschaft ausgebildet, in die das Ausstoßventil und/oder der Sauganschluss eingeführt werden. Wenn das erfindungsgemäße Prothesenschaftsystem für eine untere Extremität ausgebildet ist, kann der Innenraum zwischen Innenschaft und Extremitätenstumpf beim Gehen evakuiert werden, indem der Amputationsstumpf die im Innenraum befindliche Luft beim Auftreten mit dem Fuß des versorgten Beines auf den Boden aus dem Ausstoßventil herausstößt. An einen Sauganschluss kann eine manuelle oder elektrische Pumpe angeschlossen werden, mittels derer sich der Innenraum bereits direkt nach dem Einstieg in die vor dem Nutzen der Prothese evakuieren lässt. Bei der Nutzung der Prothese kann der Innenraum durch eine Pumpe zusätzlich evakuiert werden. Lediglich ein einziges Ausstoßventil und/oder einen Sauganschluss kann das erfindungsgemäße Prothesenschaftsystem aufweisen, es können jedoch auch mehrere Ausstoßventile und/oder Sauganschlüsse in dem Prothesenschaftsystem Anwendung finden.

In einer Weiterbildung der Erfindung sind der Innenschaft und der Außenschaft lösbar und hubfrei, insbesondere durch eine Schraubverbindung mit in den Innenschaft integrierten Setzmuttern, miteinander verbunden. Hubfrei meint, dass der Innenschaft und der Außenschaft beim Bewegen des mit dem Prothesenschaftsystem versorgten Extremitätenstumpfes miteinander und ohne bzw. mit möglichst geringer Relativbewegung zueinander bewegt werden.

In einer Weiterbildung der Erfindung ist der Außenschaftrand in einem ventralen Bereich aus einem flexiblen und unelastischen Material, bevorzugt aus einem faserverstärkten Kunststoff, insbesondere aus einem mit einer hochfesten Kohlenstofffaser, Aramidfaser oder Polyethylenfaser, gebildet, wobei das flexible und unelastische Material an eine Form eines Extremitätenstumpfes eines Trägers des Prothesenschaftes angepasst ist. Dies bedeutet nicht zwangsläufig eine individuelle Anpassung an jeden einzelnen Prothesenträger, sondern auch eine standardmäßige Anpassung an die erwartete Form des Extremitätenstumpfes eines Prothesenträgers.

Der Innenschaft kann zumindest bereichsweise elastisch ausgebildet sein oder zumindest bereichsweise aus einem elastischen Material bestehen. Dadurch ist es möglich, Volumenänderungen auszugleichen oder Druckspitzen abzubauen.

Ausführungsbeispiele der Erfindung werden nachfolgend anhand der beigefügten Figuren erläutert. Es zeigen:
- Figur 1 -: eine Frontalansicht eines Ausführungsbeispiels eines erfindungsgemäßen Prothesenschaftsystems,
- Figur 2 -: eine laterale Ansicht eines Ausführungsbeispiels eines erfindungsgemäßen Prothesenschaftsystems,
- Figur 3 -: eine frontale Querschnittsansicht eines Innenschaftes eines Ausführungsbeispieles des erfindungsgemäßen Prothesenschaftsystem s;
- Figur 4 -: eine mediale Querschnittsansicht eines Innenschaftes eines Ausführungsbeispieles des erfindungsgemäßen Prothesenschaftsystems;
- Figur 5 -: eine Frontalansicht eines Oberschenkelschaftes;
- Figur 6 -: eine angenäherte Lateral-Medialansicht eines Oberschenkelschaftes gemäß Figur 5,
- Figur 7 -: eine Teilschnittdarstellung eines Prothesenschaftsystems;
- Figur 8 -: eine perspektivische Darstellung einer Variante eines Innenschaftes; sowie
- Figur 9 -: eine Schnittdarstellung gemäß Figur 8.

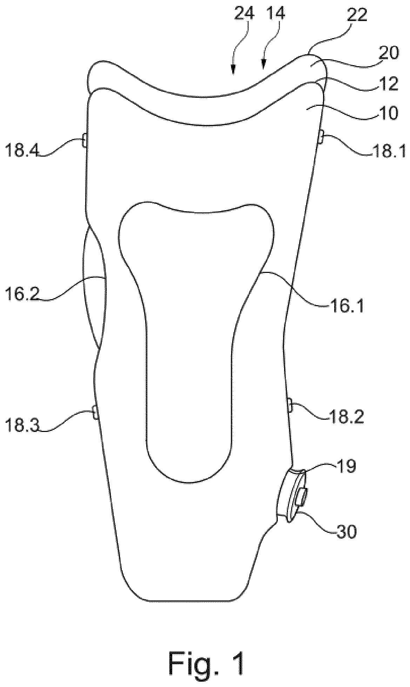

Figur 1 zeigt ein Ausführungsbeispiel eines erfindungsgemäßen Prothesenschaftsystems in einer Frontalansicht. Bei dem vorliegenden Prothesenschaftsystem handelt es sich um ein Prothesenschaftsystem für einen rechten Unterschenkel eines transtibial amputierten Patienten. Das Prothesenschaftsystem weist einen starren Außenschaft 10 und einen flexiblen Innenschaft 20 auf. Der flexible Innenschaft 20 ist in dem starren Außenschaft 10 angeordnet, wobei der Innenschaft 20 in proximaler Richtung aus dem Außenschaft 10 herausragt. In Richtung einer proximalen Außenschaftöffnung 14 weist der Außenschaft 10 einen Außenschaftrand 12 auf. In Richtung einer proximalen Innenschaftöffnung 24 weist der Innenschaft 20 einen Innenschaftrand 22 auf. Die Außenschaftöffnung 14 und die Innenschaftöffnung 24 dienen der Aufnahme des rechten Unterschenkelstumpfes des Patienten.

Zwei Fenster 16.1 und 16.2 sind in dem Außenschaft 10 ausgebildet und werden von dem Innenschaft 20 ausgefüllt. Insofern ist in den Fenstern 16.1 und 16.2 eine Außenseite des Innenschaftes 20 zu erkennen. Das frontale Fenster 16.1 ist in Form, Anordnung und Größe für eine Entlastung der Tibia des Patienten ausgebildet. Das laterale Fenster 16.2 ist in Form, Anordnung und Größe für eine Entlastung des Caput Fibulae des Patienten ausgebildet. Dabei kann es sich um individuelle Anpassungen der Form, Anordnung und Größe der Fenster an die Anatomie beispielsweise des jeweiligen Prothesenträgers, eines durchschnittlichen Menschen oder für unterschiedliche Körpergrößen oder Geschlechter handeln. Der Außenschaft 10 ist mittels Schrauben 18.1, 18.2, 18.3 und 18.4 mit dem Innenschaft 20 verbunden. Die Schrauben 18.1 und 18.2 liegen auf einer medialen Seite des Prothesenschaftsystemes und die Schrauben 18.3 und 18.4 liegen auf einer lateralen Seite des Prothesenschaftsystemes. Anzahl und Anordnung der Schrauben 18.1 bis 18.4 sind hier lediglich beispielhaft dargestellt. Es können auch mehr oder weniger Schrauben ausgebildet sein und/oder andere Positionen gewählt werden. Insbesondere kann für das Verbinden des Außenschaftes 10 mit dem Innenschaft 20 eine andere Verbindungstechnik als eine Verschraubung gewählt werden, beispielsweise eine lösbare formschlüssige Verbindung über Clips, Klettverschlüsse, Verhakungsbereiche oder andere, reversible Verbindungssysteme.

In dem Außenschaft 10 ist medial eine Außenschaftausnehmung 19 zur Aufnahme eines Ausstoßventils 30 angeordnet. Alternativ oder zusätzlich kann ein Sauganschluss in der Außenschaftausnehmung 19 angeordnet werden oder zumindest eine weitere Außenschaftausnehmung 19 in dem Außenschaft 10 ausgebildet sein, um ein zweites Ausstoßventil einzusetzen oder ein Pumpe anzuschließen.

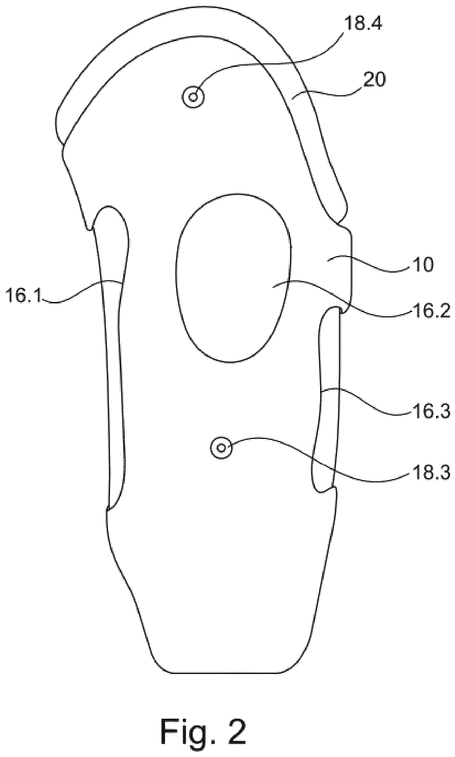

Figur 2 zeigt eine laterale Ansicht des Prothesenschaftsystemes aus Figur 1. Besonders gut zu erkennen ist hier das laterale Fenster 16.2 zur Entlastung des Caput Fibulae. Auch die dorsale Anordnung des Fensters 16.3 zur Entlastung des distalen Wadenbereiches ist sichtbar. Die lateralen Schrauben 18.3 und 18.4, die den Außenschaft 10 und den Innenschaft 20 auf dieser Seite des Prothesenschaftsystems miteinander verbinden, sind gut zu erkennen. Ein weiteres Fenster 16.1 ist auf der ventralen oder frontalen Seite des Außenschaftes 10 und entlastet einen Bereich der Tibia. Der Innenschaft 20 füllt auch hier sämtliche Fenster 16.1, 16.2 und 16.3 aus, so dass in den Fenstern 16.1, 16.2 und 16.3 jeweils die Außenseite des Innenschaftes 20 sichtbar ist. Die Konturen des Außenschaftes 10 und des Innenschaftes 20 sind zueinander ähnlich, insbesondere die proximalen Ränder 12, 22 weisen einen ähnlichen Verlauf auf und sind dorsal und ventral auf unterschiedlichen Niveaus angeordnet, während die medialen und lateralen Bereiche gebogen und proximal zu den ventralen und dorsalen Abschnitten ausgebildet sind. Dadurch wird eine erhöhte seitliche Stabilität bei einer gleichzeitigen hohen Beweglichkeit des verbliebenen Gelenkes erreicht.

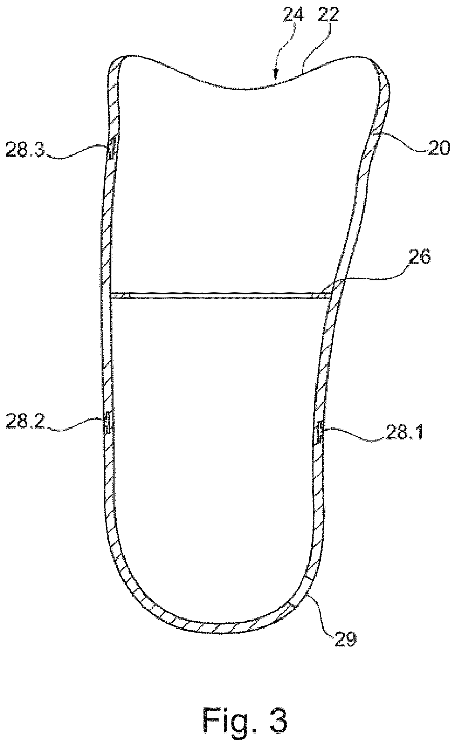

Figur 3 zeigt eine frontale Querschnittsansicht eines Innenschaftes 20 nach einem Ausführungsbeispiel des erfindungsgemäßen Prothesenschaftsystemes. Zu erkennen ist hier eine an den Innenschaft 20 angeformte, umlaufende Dichtlippe 26. Die Dichtlippe 26 ist in einer im Wesentlichen orthogonal zur Längserstreckung in Proximal-Distal-Richtung des Innenschaftes 20 verlaufenden Ebene angeordnet.

Der flexible Innenschaft 20 weist in diesem Ausführungsbeispiel eine Materialstärke in Medial-Lateral-Richtung von 3 mm - 4 mm auf. Die umlaufende Dichtlippe 26 weist eine Materialstärke von 0,8 mm in Proximal-Distal-Richtung auf. Die umlaufende Dichtlippe 26 ist näher an der proximalen Innenschaftöffnung 24 als an einem distalen Endabschnitt des Innenschaftes 20 angeordnet. An der medialen Seite des Innenschaftes 20 ist eine mediale Setzmutter 28.1 angeordnet. An der lateralen Seite des Innenschaftes 20 sind zwei laterale Setzmuttern 28.2 und 28.3 angeordnet. Die Setzmuttern 28.1, 28.2, 28.3 sind hier nur beispielsweise in ihrer Anzahl und in ihrer Anordnung gewählt. Die Setzmuttern 28.1, 28.2 und 28.3 dienen der Verschraubung mit dem hier nicht dargestellten Außenschaft 10 des Prothesenschaftsystemes. In einem distal-medial befindlichen Abschnitt des Innenschaftes 20 ist eine Innenschaftausnehmung 29 ausgebildet. Die Innenschaftausnehmung 29 dient der Aufnahme beispielsweise eines hier nicht dargestellten Ausstoßventiles 30. Alternativ kann aber auch ein Sauganschluss oder ein anderes Mittel zur Erzeugung eines Unterdruckes in dem Innenschaft 20 angeordnet werden.

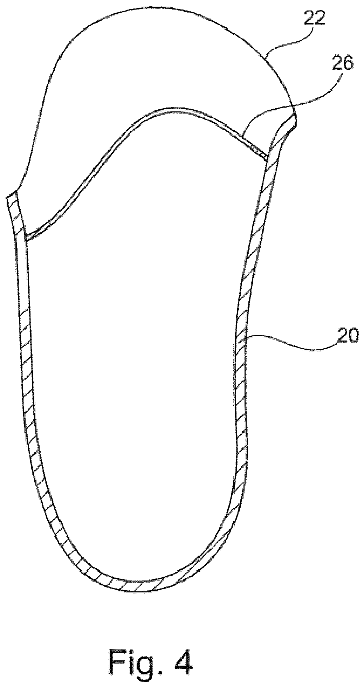

Figur 4 zeigt eine mediale Querschnittsansicht eines Innenschaftes 20 nach einem anderen Ausführungsbeispiel des erfindungsgemäßen Prothesenschaftsystems. Die Kontur des Innenschaftrandes 22 verläuft entlang einer Raumkurve von einem unteren, ventralen dorsalen Abschnitt zu einem oberen, dorsalen ventralen Abschnitt, wobei sie in einem lateralen und/ oder medialen Abschnitt dazwischen einen Hochpunkt aufweist. Die Dichtlippe 26 verläuft im Wesentlichen vollständig in einem gleichen Abstand zu der Kontur des Innenschaftrandes 22. Anders als in Figur 3 ist die Dichtlippe 26 nicht in einer Ebene angeordnet, sondern verläuft mit der Kontur des Innenschaftrandes 22 entlang einer Raumkurve. Alternativ ist vorgesehen sein, dass die Dichtlippe 26 nur teilweise parallel bzw. annähernd parallel zu der Kontur des Innenschaftrandes 22 oder einer Kontur des hier nicht dargestellten Außenschaftrandes 12 des Außenschaftes 10 verläuft und in den übrigen Bereichen einen Verlauf hat, der den Dichterfordernissen oder Komfortwünschen des Patienten Rechnung trägt.

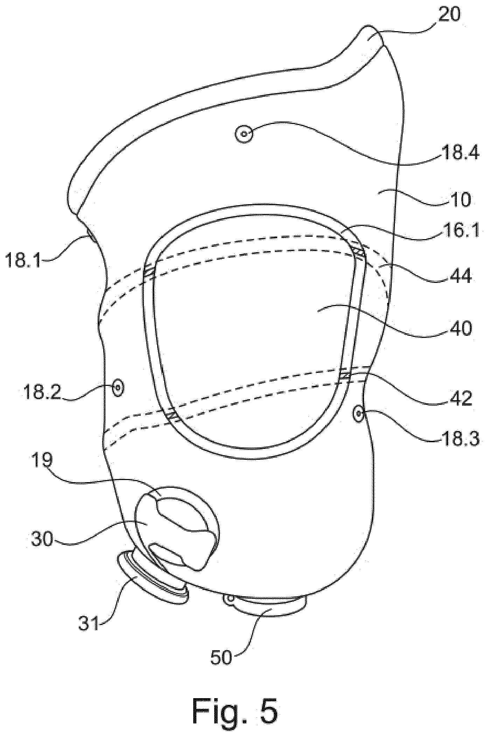

In der Figur 5 ist eine Frontalansicht eines Prothesenschaftsystems in Gestalt eines Oberschenkelschaftes mit einem Außenschaft 10 und einem darin angeordneten Innenschaft 20 dargestellt. Die Schrauben 18.1, 18.2, 18.3, 18.4 sind medial und lateral an dem Außenschaft 10 angeordnet und befestigen den elastischen Innenschaft 20 reversibel an dem Außenschaft 10. Innerhalb des Außenschaftes 10, der als formstabiles Bauteil, beispielsweise aus einem Kunststoff oder einem faserverstärkten Verbundwerkstoff, ausgebildet sein kann, ist ein frontales Fenster 16.1 ausgebildet oder angeordnet, in dem ein Einsatz 40 angeordnet ist. Die Kontur des Einsatzes 40 entspricht im Wesentlichen der Kontur des Fensters 16.1, lässt jedoch einen Freiraum zwischen der Außenkontur des Einsatzes 40 und der Innenkontur des Fensters 16.1. Der Einsatz 40 ist innerhalb des Fensters 16.1 verstellbar an dem Außenschaft 10 befestigt. Die Befestigung erfolgt durch ein Spannelement 42, das als Kabel, Gurt oder Schnursystem ausgebildet sein kann. Das Spannelement 42 kann über ein Wickelsystem oder eine Spanneinrichtung 31 in der effektiven Länge vergrößert oder verkleinert werden, so dass bei einer gekrümmten Kontur des Außenschaftes 10 eine radial nach innen gerichtete Verlagerung des Einsatzes 40 bei einer Verkürzung des Spannelementes 42 erfolgt. Wird die Spanneinrichtung 31 gelöst und das Spannelement 42 entspannt, kann der Einsatz 40 innerhalb des Fensters 16.1 bewegt werden, insbesondere radial nach außen, um eine Volumenvergrößerung innerhalb des Innenschaftes 20 und des Außenschaftes 10 zu ermöglichen. Das Spannelement 42 ist in Kanälen 44 innerhalb des Außenschaftes 10 und des Einsatzes 40 geführt. Alternativ zu einer vollständigen Führung des Spannelementes 42 in geschlossenen Kanälen 44 können diese auch teilweise offen ausgebildet sein, insbesondere nach außen offen und mit einem Füllmaterial oder einer Polsterung abgedeckt sein.

Innerhalb des Außenschaftes 10 ist eine Außenschaftausnehmung 19 angeordnet, in der das Ausstoßventil 30 angeordnet ist. Unterhalb des Ausstoßventils 30 ist die Spanneinrichtung 31 an dem Außenschaft 10 befestigt, die als Aufwickeleinrichtung für das Spannelement 42 dient. Die Spanneinrichtung 31 kann in zwei Richtungen gedreht werden, um einerseits das Spannelement 42 aufzuwickeln und andererseits bei einer entgegengesetzten Drehrichtung das Spannelement 42 zu verlängern, damit der Einsatz 40 gelockert werden kann. Weiterhin kann die Spanneinrichtung 31 vollständig entkoppelt werden.

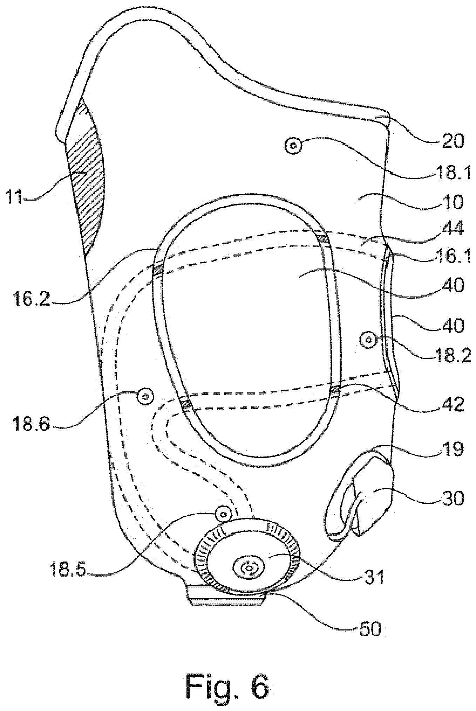

Figur 6 zeigt eine Lateralansicht des Prothesenschaftsystems mit dem Außenschaft 10 und dem darin reversibel festgelegten Innenschaft 20 zur Aufnahme eines Oberschenkelstumpfes. Neben der frontalen Ausnehmung oder dem frontalen Fenster 16.1 und dem darin angeordneten Einsatz 40 ist eine laterale mediale Ausnehmung 16.2 in dem Außenschaft 10 ausgebildet, in dem ein zweiter Einsatz 40 gelagert ist. Auch hier ist die Außenkontur des zweiten Einsatzes 40 korrespondierend zu der Innenkontur des zweiten Fensters 16.2 ausgebildet, wobei ein Spalt zwischen dem Einsatz 40 und dem Rand des Fensters 16.2 vorhanden ist. Das Spannelement 42 ist in den Kanälen 44 in dem Einsatz 40 und dem Außenschaft 10 geführt. In dem dargestellten Ausführungsbeispiel verläuft das Spannelement 42 umlaufend von der gegenüberliegenden, nicht dargestellten medialen lateralen Seite über die frontale Seite zur lateralen medialen Seite des Prothesenschaftsystems und wird mit den beiden freien Enden an der Spanneinrichtung 31 zusammengeführt. Durch Drehen in die eine oder andere Richtung, wie durch die Pfeile angedeutet, kann das Spannelement 42 durch die Spanneinrichtung 31 verkürzt oder verlängert werden.

Auf der rückwärtigen dorsalen Seite des Außenschaftes 10 ist ein elastischer flexibler Bereich 11 ausgebildet, der weicher flexibler und nachgiebiger als das übrige Material des Außenschaftes 10 ist. Dadurch wird beispielsweise das Sitzen erleichtert. Zusätzliche Schrauben 18.5, 18.6 sind im rückwärtigen Bereich des Außenschaftes 10 angeordnet und verbinden den Innenschaft 20 mit dem Außenschaft 10.

An dem distalen Ende des Außenschaftes 10 ist eine Anschlusseinrichtung oder Konnektor 50 zum Befestigen des Prothesenschaftsystems an einem nicht dargestellten Prothesenkniegelenk angeordnet oder ausgebildet. Die Anschlusseinrichtung 50 ist insbesondere als eine Adapteraufnahme für einen Pyramidenadapter ausgebildet.

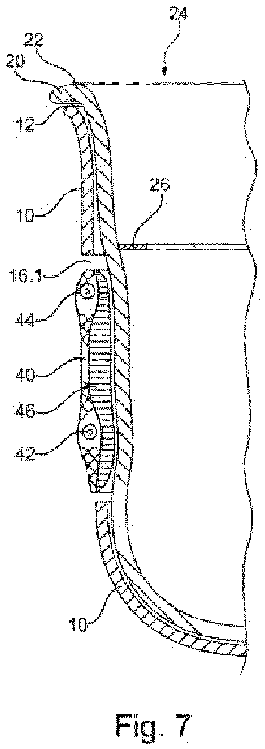

Figur 7 zeigt eine Teilschnittdarstellung des Prothesenschaftsystems mit einem formstabilen, im Wesentlichen starren Außenschaft 10, der zumindest bereichsweise umlaufend ausgebildet ist. Innerhalb des Außenschaftes 10 ist der flexible, insbesondere elastische Innenschaft 20 angeordnet. An dem proximalen Ende ist der Innenschaftrand 22 ausgebildet, der sich über den Außenschaftrand 12 radial nach außen erstreckt, um eine Abpolsterung des Außenschaftes 10 im proximalen Endbereich zu ermöglichen. In dem Außenschaft 10 ist ein Fenster 16.1 ausgebildet, in dem ein Einsatz 40 angeordnet ist. Innerhalb des Einsatzes 40 sind Kanäle 44 angeordnet, in denen das Spannelement 42 verschieblich oder verlagerbar geführt ist. Das Spannelement 42 ist beispielsweise als Schnur, Litze, Gurt oder Kabel ausgebildet, alternative Zugelemente können ebenfalls als Spannelement 42 eingesetzt werden. Auf der Innenseite des Einsatzes 40, also auf derjenigen Seite, die dem Innenschaft 20 zugewandt ist, ist ein Polster 46 angeordnet, das in den Innenraum des Außenschaftes 10 hineinragt. Eine Verlängerung der Außenwand des Außenschaftes 10 hätte einen im Wesentlichen geradlinigen Verlauf und würde die Öffnung 16.1 überbrücken. Das Polster 46 ragt radial nach innen in den Innenraum des Außenschaftes 10 hinein und drückt, wie in der Figur 7 dargestellt, den Innenschaft 20 radial nach innen in Richtung auf den nicht dargestellten Oberschenkelstumpf. Wird durch die Spanneinrichtung 31 das Spannelement 42 aufgewickelt und in der effektiven Länge verkürzt, spannt das Spannelement 42 zwischen den Kanalöffnungen in dem Außenschaft 10 und neigt dazu, eine Gerade auszubilden. Dadurch wird der Einsatz 40 nach innen bewegt, so dass eine Volumenverringerung des Innenschaftes 20 erfolgt. Dadurch wird der Innenschaft 20 an den nicht dargestellten Stumpf angepresst. Dies kann beispielsweise zur Anpassung an eine Volumenschwankung des Stumpfes, an ein geändertes Komfortbedürfnis oder aber an ein geändertes Aktivitätsniveau erfolgen. Wird beispielsweise eine höhere Gehgeschwindigkeit angestrebt, erhöhen sich die Fliehkräfte, so dass ein Spannen des Spannelementes 42 und ein Anpressen des Einsatzes 40 mit dem Polster 46 in Richtung auf den Oberschenkelstumpf als zusätzliche Sicherung empfunden wird.

Radial nach innen ragt von dem Innenschaft 20 die Dichtlippe 26, die sich in dem dargestellten Ausführungsbeispiel im Wesentlichen waagerecht erstreckt. Grundsätzlich sind auch andere Verläufe der Dichtlippe 26 radial innen innerhalb des Innenschaftes 20 vorgesehen und möglich. Beispielsweise kann die Dichtlippe 26 der Kontur des Außenschaftrandes 12 folgen. Ebenso ist es möglich, dass die Dichtlippe 26 quer über ein Fenster 16.1 verläuft, statt wie im dargestellten Ausführungsbeispiel oberhalb des Fensters 16.1. Die Dichtlippe 26 kann um ein Fenster 16.1 herum angeordnet sein. Das Polster 46 kann elastisch ausgebildet sein, ebenso kann es als aufblasbares und im Volumen veränderbares Kissen ausgebildet sein. Die Innenkontur, also diejenige Kontur, die in Richtung auf den Innenschaft 20 gerichtet ist, kann eine Pelottenform aufweisen. Eine an die Körperkontur angepasste Form des Polsters 46 ist alternativ vorgesehen.

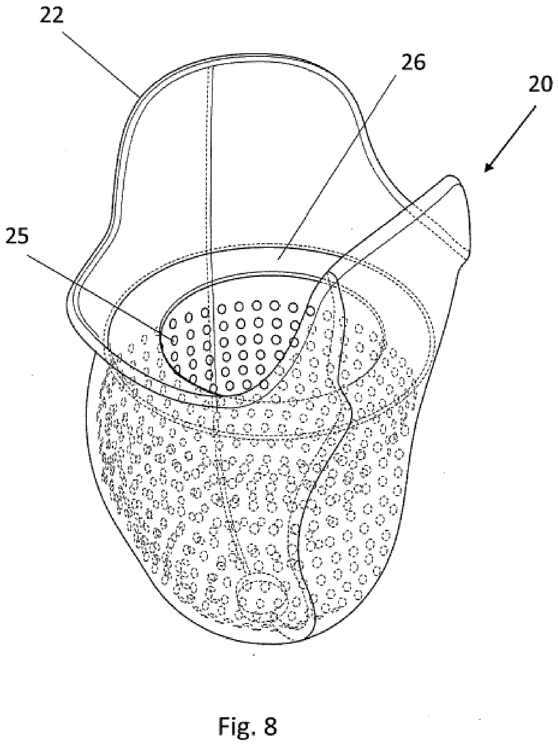

In der Figur 8 ist in einer perspektivischen Darstellung eine weitere Variante des Innenschaftes 20 dargestellt, der geschlossenwandig ausgebildet ist und einen proximalen Rand 22 aufweist, der an der lateralen Seite erhöht ist, um eine großflächige und sichere Anlage an dem Patienten zu ermöglichen. Innerhalb des durch den Innenschaft 20 ausgebildeten Hohlraumes ist eine umlaufende Dichtung 26 angeformt, die radial nach innen ragt und sich im angelegten Zustand um den nicht dargestellten Stumpf herum legt. Die Dichtlippe 26 kann von radial außen nach radial innen mit einem sich verringernden Querschnitt oder einer verringernden Materialstärke ausgebildet sein, um eine besonders flexible und ganzflächige Anlage an dem Stumpf des Patienten zu ermöglichen.

Distal zu der Dichtlippe 26, die im dargestellten Ausführungsbeispiel in einer Ebene angeordnet ist, sind an der Innenoberfläche Noppen 25 oder Vertiefungen ausgebildet, um die Innenoberfläche des Innenschaftes zu vergrößern. Insbesondere durch die Ausgestaltung von Noppen 25 an der Innenoberfläche ist es möglich, eine vergrößerte Anlageoberfläche und damit eine verbesserte Anhaftung an dem Stumpf zu erreichen.

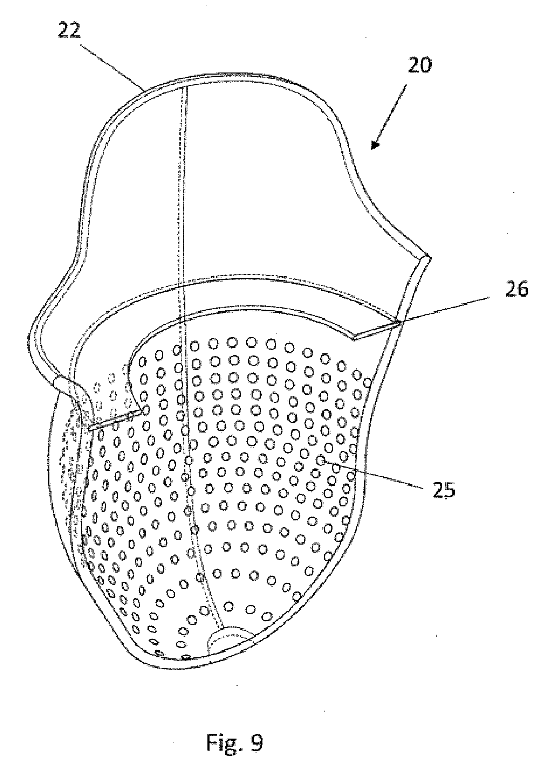

Figur 9 zeigt die Ausgestaltung des Innenschaftes 20 in einer Schnittdarstellung.

Die umlaufende Dichtlippe 26 ist in dem dargestellten Ausführungsbeispiel als separate, kreisringförmige Dichtlippe 26 ausgebildet, die in einer vorgefertigten Nut innerhalb des Innenschaftes 20 eingesetzt und daran gesichert ist, beispielsweise verklebt, eingegossen oder verschweißt. Alternativ dazu ist es möglich, die Dichtlippe 26 einstückig mit dem übrigen Innenschaft 20 auszubilden. Ebenso können die Noppen 22 einstückig mit dem Innenschaft 20 ausgebildet oder nachträglich auf der Innenseite angeordnet und befestigt werden, beispielsweise durch Verkleben, Angießen oder Verschweißen.

Sofern eine Oberflächenvergrößerung durch Einarbeitung von Vertiefungen der Wand des Innenschaftes 20 erfolgen soll, können diese während des Urformprozesses oder auch nachträglich ausgebildet werden. Bei einer Ausgestaltung von Vorsprüngen auf der Innenseite können diese Noppen 25 im Wesentlichen parallel zu der Dichtlippe 26 verlaufenden, umlaufenden Reihen angeordnet oder ausgebildet sein. Die Reihen von Vorsprüngen oder Noppen 25 können gleichmäßig zueinander beabstandet in Proximal-Distal-Richtung angeordnet oder ausgebildet sein. Ebenso ist es möglich, Reihen in Proximal-Distal-Richtung orientiert und in Umfangsrichtung nebeneinander anzuordnen. Neben einer einzelnen Ausgestaltung der Vorsprünge als Noppen können diese auch als Rillen, Nuten oder vorstehende Rippen ausgebildet sein. Ebenso ist es möglich, die nach innen abstehenden Vorsprünge unterschiedlich auszubilden, beispielsweise als ringförmige, riemenartige Vorsprünge in Verbindung mit Noppen. Das Material der Vorsprünge oder Noppen 25 kann mit dem Material des übrigen Innenschaftes 20 identisch oder insbesondere weicher sein, um einen angenehmen Tragekomfort zu erreichen.

### Bezugszeichenliste

- 10: Außenschaft
- 11: elastischer flexibler Bereich
- 12: Außenschaftrand
- 14: Außenschaftöffnung
- 16.1 ... 16.3: Fenster
- 18.1 ... 18.4: Schrauben
- 19: Außenschaftausnehmung
- 20: Innenschaft
- 22: Innenschaftrand
- 24: Innenschaftöffnung
- 25: Noppen
- 26: Dichtlippe
- 28.1 ... 28.3: Setzmuttern
- 29: Innenschaftausnehmung
- 30: Ausstoßventil
- 31: Spanneinrichtung
- 40: Einsatz
- 42: Spannelement
- 44: Kanal
- 46: Polster
- 50: Anschlusseinrichtung

## Patentansprüche

1. Prothesenschaftsystem mit einem starren Außenschaft (10), der eine proximale Außenschaftöffnung (14) zur Aufnahme eines Extremitätenstumpfes eines Trägers des Prothesenschaftsystemes aufweist, und einem flexiblen Innenschaft (20), der mit dem Außenschaft (10) verbunden ist und eine proximale Innenschaftöffnung (24) zur Aufnahme des Extremitätenstumpfes aufweist, **dadurch gekennzeichnet, dass** zumindest eine Dichtlippe (26) an einer dem Extremitätenstumpf zugewandten Innenseite des Innenschaftes (20) angeordnet ist.

2. Prothesenschaftsystem nach Anspruch 1, **dadurch gekennzeichnet, dass** die Dichtlippe als eine umlaufende Dichtlippe (26) ausgebildet ist, die entlang eines gesamten Innenumfanges der Innenseite des Innenschaftes (20) angeordnet ist.

3. Prothesenschaftsystem nach Anspruch 1 oder 2, **dadurch gekennzeichnet, dass** in dem Außenschaft (10) zumindest ein Fenster (16.1, 16.2, 16.3) ausgebildet ist.

4. Prothesenschaftsystem nach Anspruch 3, **dadurch gekennzeichnet, dass** die Dichtlippe (26) zumindest teilweise in einem Bereich des Innenschaftes (20) angeordnet ist, der von dem Fenster (16.1, 16.2, 16.3) überdeckt ist.

5. Prothesenschaftsystem nach Anspruch 3 oder 4, **dadurch gekennzeichnet, dass** der Außenschaft (10) als Unterschenkelschaft ausgebildet ist und ein Fenster (16.1, 16.2, 16.3) in einem frontalen Schienbeinbereich und/oder im Bereich eines Wadenbeinköpfchens und/oder in einem distalen Wadenbeinbereich angeordnet ist.

6. Prothesenschaftsystem nach einem der Ansprüche 3 bis 5, **dadurch gekennzeichnet, dass** in zumindest einem der Fenster (16.1, 16.2, 16.3) ein verstellbarer Einsatz (40) angeordnet ist, der an dem Außenschaft (10) befestigt ist.

7. Prothesenschaftsystem nach einem der voranstehenden Ansprüche, **dadurch gekennzeichnet, dass** die Dichtlippe (26) näher an der proximalen Innenschaftöffnung (24) als einem distalen Ende des Innenschaftes (20) angeordnet ist.

8. Prothesenschaftsystem nach einem der voranstehenden Ansprüche, **dadurch gekennzeichnet, dass** die Dichtlippe (26) in einer Ebene angeordnet ist.

9. Prothesenschaftsystem nach einem der voranstehenden Ansprüche, **dadurch gekennzeichnet, dass** die Dichtlippe (26) zumindest teilweise parallel zu einer Kontur eines die Innenschaftöffnung (24) umgebenden Innenschaftrandes (22) oder einer Kontur eines die Außenschaftöffnung (14) umgebenden Außenschaftrandes (12) angeordnet ist.

10. Prothesenschaftsystem nach einem der voranstehenden Ansprüche, **dadurch gekennzeichnet, dass** die Dichtlippe (26) stoffschlüssig mit dem Innenschaft (20) verbunden oder daran angeformt ist und/oder die Dichtlippe (26) und der Innenschaft (20) aus demselben Material bestehen.

11. Prothesenschaftsystem nach einem der voranstehenden Ansprüche, **dadurch gekennzeichnet, dass** das Prothesenschaftsystem ein Ausstoßventil (30) und/oder einen Sauganschluss zur Regulierung eines Unterdruckes zwischen dem Extremitätenstumpf und dem Innenschaft (20) aufweist.

12. Prothesenschaftsystem nach einem der voranstehenden Ansprüche, **dadurch gekennzeichnet, dass** der Innenschaft (20) und der Außenschaft (10) lösbar und hubfrei, insbesondere durch eine Schraubverbindung mit in den Innenschaft (20) integrierten Setzmuttern (8.1, 28.2, 28.3), miteinander verbunden sind.

13. Prothesenschaftsystem nach einem der voranstehenden Ansprüche, **dadurch gekennzeichnet, dass** der Innenschaft (20) zumindest bereichsweise elastisch ausgebildet ist oder zumindest bereichsweise aus einem elastischen Material besteht.

## Claims

1. A prosthesis socket system with a rigid outer socket (10), which has a proximal outer socket opening (14) for receiving an extremity stump of a wearer of the prosthesis socket system, and with a flexible inner socket (20), which is connected to the outer socket (10) and has a proximal inner socket opening (24) for receiving the extremity stump, **characterized in that** at least one sealing lip (26) is arranged on an inner face of the inner socket (20) directed toward the extremity stump.

2. The prosthesis socket system as claimed in claim 1, **characterized in that** the sealing lip is configured as a peripheral sealing lip (26), which is arranged along a complete inner circumference of the inner face of the inner socket (20).

3. The prosthesis socket system as claimed in claim 1 or 2, **characterized in that** at least one window (16.1, 16.2, 16.3) is formed in the outer socket (10).

4. The prosthesis socket system as claimed in claim 3, **characterized in that** the sealing lip (26) is arranged at least partially in a region of the inner socket (20) that is covered by the window (16.1, 16.2, 16.3).

5. The prosthesis socket system as claimed in claim 3 or 4, **characterized in that** the outer socket (10) is configured as a lower-leg socket, and a window (16.1, 16.2, 16.3) is arranged in a frontal shin region and/or in the region of a head of a fibula and/or in a distal calf region.

6. The prosthesis socket system as claimed in one of claims 3 through 5, **characterized in that** an adjustable insert (40), which is fastened to the outer socket (10), is arranged in at least one of the windows (16.1, 16.2, 16.3).

7. The prosthesis socket system as claimed in one of the preceding claims, **characterized in that** the sealing lip (26) is arranged closer to the proximal inner socket opening (24) than to a distal end of the inner socket (20).

8. The prosthesis socket system as claimed in one of the preceding claims, **characterized in that** the sealing lip (26) is arranged in one plane.

9. The prosthesis socket system as claimed in one of the preceding claims, **characterized in that** the sealing lip (26) is arranged at least partially parallel to a contour of an inner socket edge (22) surrounding the inner socket opening (24) or to a contour of an outer socket edge (12) surrounding the outer socket opening (14).

10. The prosthesis socket system as claimed in one of the preceding claims, **characterized in that** the sealing lip (26) is cohesively bonded to the inner socket (20) or formed integrally thereon, and/or the sealing lip (26) and the inner socket (20) are made of the same material.

11. The prosthesis socket system as claimed in one of the preceding claims, **characterized in that** the prosthesis socket system has a release valve (30) and/or a suction attachment for regulating a negative pressure between the extremity stump and the inner socket (20).

12. The prosthesis socket system as claimed in one of the preceding claims, **characterized in that** the inner socket (20) and the outer socket (10) are connected to each other releasably and in a manner free of pistoning, in particular by a screw connection with locking nuts (8.1, 28.2, 28.3) integrated in the inner socket (20).

13. The prosthesis socket system as claimed in one of the preceding claims, **characterized in that** the inner socket (20) is elastic at least in some regions or is made of an elastic material at least in some regions.

## Revendications

1. Système d'emboîtures prothétique comprenant une emboîture extérieure rigide (10) qui présente une ouverture d'emboîture extérieure (14) proximale pour recevoir un moignon de membre d'un porteur du système d'emboîtures prothétique, et comprenant une emboîture intérieure (20) flexible qui est reliée à l'emboîture extérieure (10) et qui présente une ouverture d'emboîture intérieure (24) proximale pour recevoir le moignon de membre, **caractérisé en ce qu'**au moins une lèvre d'étanchéité (26) est disposée sur un côté intérieur de l'emboîture intérieure (20) tourné vers le moignon de membre.

2. Système d'emboîtures prothétique selon la revendication 1, **caractérisé en ce que** la lèvre d'étanchéité est réalisée sous forme de lèvre d'étanchéité circonférentielle (26) qui est disposée le long de toute une circonférence intérieure du côté intérieur de l'emboîture intérieure (20).

3. Système d'emboîtures prothétique selon la revendication 1 ou 2, **caractérisé en ce qu'**au moins une fenêtre (16.1, 16.2, 16.3) est formée dans l'emboîture extérieure (10).

4. Système d'emboîtures prothétique selon la revendication 3, **caractérisé en ce que** la lèvre d'étanchéité (26) est disposée au moins partiellement dans une zone de l'emboîture intérieure (20) qui est recouverte par la fenêtre (16.1, 16.2, 16.3).

5. Système d'emboîtures prothétique selon la revendication 3 ou 4, **caractérisé en ce que** l'emboîture extérieure (10) est réalisée sous forme d'emboîture de jambe inférieure, et une fenêtre (16.1, 16.2, 16.3) est disposée dans une zone frontale du tibia et/ou dans la zone d'une tête de fibula et/ou dans une zone distale du fibula.

6. Système d'emboîtures prothétique selon l'une des revendications 3 à 5, **caractérisé en ce qu'**un insert réglable (40) est disposé dans l'une au moins des fenêtres (16.1, 16.2, 16.3) et est fixé à l'emboîture extérieure (10).

7. Système d'emboîtures prothétique selon l'une des revendications précédentes, **caractérisé en ce que** la lèvre d'étanchéité (26) est disposée plus près de l'ouverture d'emboîture intérieure (24) proximale que d'une extrémité distale de l'emboîture intérieure (20).

8. Système d'emboîtures prothétique selon l'une des revendications précédentes, **caractérisé en ce que** la lèvre d'étanchéité (26) est disposée dans un plan.

9. Système d'emboîtures prothétique selon l'une des revendications précédentes, **caractérisé en ce que** la lèvre d'étanchéité (26) est disposée au moins partiellement parallèlement à un contour d'une bordure d'emboîture intérieure (22) entourant l'ouverture d'emboîture intérieure (24) ou à un contour d'une bordure d'emboîture extérieure (12) entourant l'ouverture d'emboîture extérieure (14).

10. Système d'emboîtures prothétique selon l'une des revendications précédentes, **caractérisé en ce que** la lèvre d'étanchéité (26) est reliée par coopération de matière à l'emboîture intérieure (20) ou est conformée sur celle-ci, et/ou la lèvre d'étanchéité (26) et l'emboîture intérieure (20) sont constituées du même matériau.

11. Système d'emboîtures prothétique selon l'une des revendications précédentes, **caractérisé en ce que** le système d'emboîtures prothétique comprend une valve d'éjection (30) et/ou un raccord d'aspiration pour réguler une dépression entre le moignon de membre et l'emboîture intérieure (20).

12. Système d'emboîtures prothétique selon l'une des revendications précédentes, **caractérisé en ce que** l'emboîture intérieure (20) et l'emboîture extérieure (10) sont reliées l'une à l'autre de manière amovible et sans course, en particulier par une liaison vissée avec des écrous à sertir (8.1, 28.2, 28.3) intégrés dans l'emboîture intérieure (20).

13. Système d'emboîtures prothétique selon l'une des revendications précédentes, **caractérisé en ce que** l'emboîture intérieure (20) est réalisée au moins localement de façon élastique ou est constituée au moins localement d'un matériau élastique.
